# EUROPÄISCHE PATENTSCHRIFT

(11) **EP 0 982 012 B1**
(45) Veröffentlichungstag und Bekanntmachung des Hinweises auf die Patenterteilung: **15.01.2003**
(21) Anmeldenummer: 99112603.8
(22) Anmeldetag: 01.07.1999
(51) Int. Cl.: A61F 2/52

(54) **Brustprothese zum Tragen in einem Büstenhalter oder dergleichen**
Mammary prosthese for wearing in a brassiere or in such a thing
Prothèse mammaire pour poiter dans un soutien-gorge ou un autre vêtement semblable

(30) Priorität: 24.08.1998 DE 19838428
(43) Veröffentlichungstag der Anmeldung: 01.03.2000
(73) Patentinhaber: F + E Gesellschaft für Bekleidungsinnovation mbH & Co. KG, 83098 Brannenburg (DE)
(72) Erfinder: Weber-Unger, Georg, 6330 Kufstein (AT); Volk, Stephan, 83714 Miesbach (DE)
(74) Vertreter: Haug, Dietmar, Dipl.-Ing.

(56) Entgegenhaltungen:
- EP-A- 0 384 951
- EP-A- 0 824 001
- WO-A-89/05615
- WO-A-98/26735
- US-A- 5 066 302

## Beschreibung

Die Erfindung betrifft eine Brustprothese zum Tragen in einem Büstenhalter oder dergleichen, mit einem Prothesenkörper, der eine die Form einer natürlichen Brust aufweisende Vorderseite, eine mit einer Vertiefung versehene Rückseite und einen einer geschlossenen Linie folgenden Prothesenrand hat, an dem die Vorderseite und die Rückseite des Prothesenkörpers jeweils enden, wobei der Prothesenkörper eine weich-elastische Silikonkautschukmasse aufweist, die in einen Beutel aus zwei längs des Prothesenrandes dicht miteinander verbundenen, elastisch dehnbaren Kunststoffolien hohlraumfrei eingeschlossen ist.

Eine solche Brustprothese wurde z.B. in der europäischen Patentanmeldung mit der Veröffentlichungsnummer 0 657 148 A1 dargestellt und beschrieben. Bei dieser Brustprothese sind in der rückwärtigen Vertiefung des Prothesenkörpers eine oder mehrere Versteifungsrippen vorgesehen, die bei aufrechter Haltung der Prothesenträgerin vertikal und parallel zur Körperlängsachse der Trägerin verlaufen und die Funktion haben, den Prothesenkörper daran zu hindern, bei aufrechter Haltung der Prothesenträgerin unter der Wirkung der Schwerkraft zusammenzusacken. Anders ausgedrückt, sollen die Versteifungsrippen die Formstabilität der Prothese gewährleisten, wenn sich die Trägerin in aufrechter Haltung befindet. Die Versteifungsrippen der bekannten Brustprothese wirken sich aber auch formerhaltend auf den Prothesenkörper in der Liegestellung der Prothesenträgerin aus, denn sie verhindern, daß sich der Prothesenkörper unter der Wirkung der Schwerkraft in Liegestellung der Prothesenträgerin horizontal streckt. Manche Prothesenträgerinnen empfinden es als unnatürlich, wenn die Prothese die gleiche Form im Liegen wie im Stand hat. Genauer ausgedrückt, bemängeln diese Prothesenträgerinnen, daß diese Prothese nicht entsprechend den natürlichen Verhältnissen im Liegen etwas flacher als bei aufrechter Haltung ist. Besonders wenn mit der Prothese sehr weiches Brustgewebe ersetzt werden soll, wie es bei älteren Patientinnen oft der Fall ist, wird als'Nachteil gesehen, daß die Prothese im Liegen unnatürlich steil vom Körper der Trägerin wegragt.

Es ist daher die Aufgabe der Erfindung, eine Brustprothese, wie sie im Oberbegriff des Patentanspruches 1 definiert ist, derart weiterzubilden, daß sie ihre Form beim Wechsel zwischen liegender und aufrechter Haltung der Trägerin ändert, so daß die Form der Brustprothese auch in liegender Haltung der Trägerin der Form einer natürlichen Brust entspricht.

Die Aufgabe der Erfindung wird entsprechend dem kennzeichnenden Teil des Patentanspruches 1 dadurch gelöst, daß die Vertiefung mindestens eine quer zur Körperlängsachse der Trägerin verlaufende Kerbe aufweist, die von sich gegenüberliegenden Wänden eines oberen und eines unteren Teils des Prothesenkörpers und einem mittleren, den oberen Teil mit dem unteren Teil flexibel verbindenden Abschnitt begrenzt wird, der es dem oberen Teil bei aufrechter Haltung der Trägerin gestattet, durch die Wirkung der Schwerkraft relativ zu dem unteren Teil kontrolliert abzusacken, wobei das kontrollierte Absacken des oberen Teils eine elastische Biegeverformung des mittleren Abschnitts bewirkt und durch Anlage zumindest eines Teiles der sich gegenüberliegenden Wände der Kerbe begrenzt wird.

Die erfindungsgemäße Prothese nimmt in liegender Haltung der Prothesenträgerin eine relativ gestreckte flache Form ein. Richtet sich die Trägerin auf, sackt der obere Teil des Prothesenkörpers unter der Wirkung der Schwerkraft soweit nach unten, bis zumindest ein Teil der sich gegenüberliegenden Wände der Kerbe aufeinandersitzen. Die durch Absacken des oberen Teils des Prothesenkörpers bedingte Biegeverformung des mittleren Abschnitts des Prothesenkörpers sowie der Druck der aufeinandersitzenden Wände der Kerbe bewirken eine verstärkte Auswölbung der Vorderseite im unteren bis mittleren Bereich des Prothesenkörpers. Die Prothese hat dann eine Form, die der Form einer natürlichen Brust aus sehr weichem Brustgewebe entspricht. Mit dieser Form wird das Körbchen des Büstenhalters sehr gut ausgefüllt, so daß eine optimale Anpassung der Prothese an die Form des sie aufnehmenden Körbchens eines gegebenen Büstenhalters erzielt wird. Die flexible Verbindung zwischen dem oberen und unteren Teil des Prothesenkörpers führt auch zu einem ausgezeichneten Schwingungsverhalten der Prothese, das wiederum dem Schwingungsverhalten einer natürlichen Brust aus sehr weichem Gewebe entspricht. Das sich abwechselnde Strecken und Zusammensacken der Prothese vermittelt beim Laufen nicht nur ein natürliches Schwingungsverhalten, sondern es fördert auch die Be- und Entlüftung der Rückseite der Prothese, denn das sich abwechselnde Aufeinandertreffen der sich gegenüberliegenden Wände der Kerbe und Aufweiten der Kerbe bewirkt ein abwechselndes Saugen und Pumpen von Luft zwischen der Rückseite der Prothese und dem Körper der Trägerin. Befindet sich die Trägerin in liegender Stellung, streckt sich die Prothese in horizontaler Richtung parallel zu der Körperlängsachse der Trägerin, wobei diese Streckung durch das Eigengewicht der Prothese, das nun senkrecht auf den Körper der Trägerin wirkt, und die durch die Verformung des mittleren Abschnitts erzeugte Rückstellkraft bewirkt wird. Eine Folge dieser Streckung der Prothese in der Liegestellung der Trägerin ist die Abflachung der Prothese, so daß nun auch in der Liegestellung der Trägerin die Form der Prothese an die Form einer natürlichen Brust, die aus sehr weichem Brustgewebe besteht, angepaßt ist. Der mittlere, flexible Abschnitt, der den oberen Teil des Prothesenkörpers mit dem unteren Teil des Prothesenkörpers verbindet, kann mit einem Filmscharnier verglichen werden, das ein Aufund Zuklappen der Kerbe gestattet, wenn die Trägerin zwischen Liegestellung und aufrechter Haltung wechselt oder sich rasch fortbewegt. Die erfindungsgemäße Prothese zeichnet sich weiterhin durch ein geringes Gewicht aus, so daß die Prothese äußerst angenehm zu tragen ist.

Nach einer Weiterbildung der Erfindung kann die Kerbe an Stelle eines geradlinigen Verlaufes im unverformten Zustand einen bogenförmigen Verlauf haben, der die sich gegenüberliegenden Wände der Kerbe zwingt, sich beim Absacken des oberen Teils in Längsrichtung der Kerbe fortschreitend aneinanderzulegen. Dadurch wird das Absacken des oberen Teils des Prothesenkörpers stärker gedämpft als bei einer Prothese, bei der die Kerbe einen geraden Verlauf hat. Vergleicht man den mittleren Abschnitt des Prothesenkörpers mit einem Scharnier, so wirkt das Scharnier durch den bogenförmigen Verlauf der Kerbe selbsthemmend. Das Formveränderungsvermögen der Prothese kann durch eine von der Geraden abweichenden Gestaltung der Kerbe stark beeinflußt und somit an die individuellen Bedürfnisse einer Prothesenträgerin angepaßt werden.

Auch die Länge der Kerbe hat einen Einfluß auf das Formveränderungsvermögen der Prothese. Als besonders vorteilhaft hat sich erwiesen, wenn die Kerbe, in Längsrichtung gesehen, im Abstand vor dem Prothesenrand endet. Bei einer derartigen Länge der Kerbe wird verhindert, daß die seitlichen Ausläufer der Prothese zu sehr abknicken, wenn sich die Prothese im abgesackten Zustand befindet.

Das Formveränderungsvermögen der Prothese kann ferner durch die Zahl der querverlaufenden Kerben gesteuert werden. Beispielsweise kann die Vertiefung mehr als eine quer zur Körperlängsachse verlaufende Kerbe aufweisen, die bezogen auf die Körperlängsachse der Trägerin, im Abstand übereinander angeordnet sind. Bei einer derartigen Prothese ergeben sich dann mehrere in Reihe geschaltete Scharniere, die alle eine elastische Biegeverformung des Prothesenkörpers durch Schwerkrafteinwirkung im Liegen und in aufrechter Haltung der Trägerin zulassen. Die Gesamtbiegeverformung wird in einem solchen Fall auf mehr als ein Scharnier verteilt.

Das Anpassungsvermögen der Prothese an die Form der natürlichen Brust beim Liegen und in aufrechter Haltung der Trägerin wird dadurch noch verbessert, daß die Silikonkautschukmasse auf eine Weichheit mit einer Penetration zwischen 150 bis 230 eingestellt wird. Vorzugsweise besteht die Silikonkautschukmasse aus zwei additionsvernetzten Komponenten, deren Viskosität im Bereich zwischen 500 und 2500 mPa.s liegt.

Die Rückseite des Prothesenkörpers kann durch ein Stoffteil abgedeckt sein, das am Prothesenrand befestigt ist. Dieses Stoffteil verringert die Reibung zwischen der Prothese und dem Körper der Trägerin, wodurch eine Beschleunigung der Absackund Streckbewegung der Prothese erzielbar ist.

Zwei Ausführungsbeispiele der Erfindung sind in den Zeichnungen dargestellt und werden im folgenden näher beschrieben. Es zeigt
- Fig. 1: eine Draufsicht auf die Rückseite einer Brustprothese gemäß einem ersten Ausführungsbeispiel im unverformten Zustand der Prothese;
- Fig. 2: einen Querschnitt der in Fig. 1 gezeigten Brustprothese entlang der Linie II-II in Fig. 1 mit Blick in Richtung der Pfeile, wobei zusätzlich die Kontur der Prothese jeweils in Liegestellung und bei aufrechter Haltung der Prothesenträgerin angedeutet ist;
- Fig. 3: eine Draufsicht auf die Rückseite einer Brustprothese gemäß einem zweiten Ausführungsbeispiel der Erfindung im unverformten Zustand der Prothese;
- Fig. 4: einen Querschnitt der in Fig. 3 gezeigten Brustprothese entlang der Linie IV-IV in Fig. 3 mit Blick in Richtung der Pfeile, wobei zusätzlich die Kontur der Prothese jeweils in Liegestellung und bei aufrechter Haltung der Prothesenträgerin angedeutet ist; und
- Fig. 5: einen Querschnitt der in Fig. 3 gezeigten Brustprothese entlang der Linie V-V in Fig. 3 mit Blick in Richtung der Pfeile, wobei die Prothese im unverformten Zustand ist.

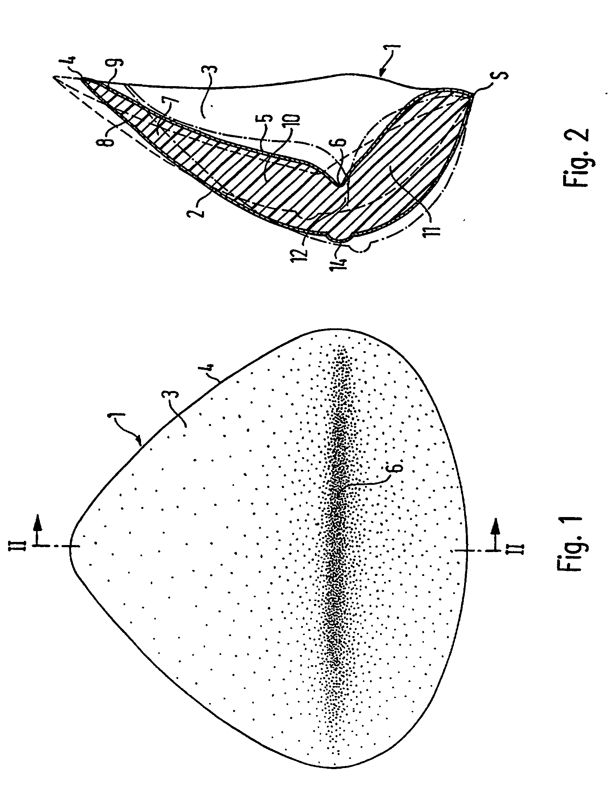

Wie in den Figuren 1 und 2 gezeigt, weist die Brustprothese gemäß einem ersten Ausführungsbeispiel einen Prothesenkörper 1 auf, der eine Vorderseite 2, eine Rückseite 3 und einen einer geschlossenen Linie folgenden Prothesenrand 4 hat, an dem die Vorderseite 2 und die Rückseite 3 jeweils enden. Die Vorderseite 2 des Prothesenkörpers 1 hat eine Form, die der Form einer natürlichen Brust entspricht. Wie unten näher erläutert ist, kann sich die Form der Prothese zwischen einer gestreckten Form und einer zusammengesackten Form in Abhängigkeit von der Haltung der Prothesenträgerin (liegend oder aufrecht) ändern. Die Prothese wird im Körbchen eines Büstenhalters getragen. Sie eignet sich auch zum Tragen im Oberteil eines Badeanzugs oder eines Bikinis. Die in Fig. 2 gestrichelt gezeigte, gestreckte Form nimmt die Prothese ein, wenn die Trägerin liegt. In der aufrechten Haltung der Trägerin sackt die Prothese zusammen, wie in Fig. 2 strichpunktiert gezeigt ist. Sowohl in der gestreckten Form wie auch in der zusammengesackten Form entspricht die Form der Vorderseite 2 der Form einer natürlichen Brust. Die Querschnittsdarstellung der Prothese in Fig. 2 zeigt die Prothese im unverformten Zustand.

Die Rückseite 3 der Prothese ist mit einer Vertiefung 5 versehen, die erfindungsgemäß eine quer zur Körperlängsachse der Trägerin verlaufende Kerbe 6 aufweist. Die Körperlängsachse der Trägerin verläuft parallel zu der in der Fig. 1 gezeichneten Schnittlinie II-II.

An der Vorderseite 2 der Prothese ist eine, eine Brustwarze 14 simulierende Noppe ausgebildet.

Der Prothesenkörper 1 setzt sich aus einer weich-elastischen Silikonkautschukmasse 7 und einem Beutel aus zwei längs des Prothesenrandes 4 dicht miteinander verschweißten, elastisch dehnbaren Kunststoffolien 8, 9 zusammen, wobei die Silikonkautschukmasse 7 in den Beutel hohlraumfrei eingeschlossen ist. Vorzugsweise sind die beiden Kunststoffolien 8, 9 Polyurethanfolien.
Die Kerbe 6 wird von den sich gegenüberliegenden Wänden eines oberen Prothesenkörperteiles 10 und eines unteren Prothesenkörperteiles 11 sowie einem mittleren Abschnitt 12 begrenzt, der den oberen Teil 10 und den unteren Teil 11 des Prothesenkörpers 1 flexibel miteinander verbindet.

Wechselt die Trägerin ihre Position von der Liegestellung in eine aufrechte Haltung, ändert die Prothese ihre Form von der in Fig. 2 gestrichelt dargestellten, gestreckten Form in die in Fig. 2 strichpunktiert dargestellte, zusammengesackte Form. Genauer ausgedrückt, sackt beim Wechsel von der Liegestellung in die aufrechte Haltung der obere Teil 10 unter der Wirkung der Schwerkraft im Körbchen des Büstenhalters nach unten ab, wobei das Absacken des oberen Teils 10 eine elastische Biegeverformung des mittleren Abschnitts 12 bewirkt. Die Absackbewegung des oberen Teils 10 wird gestoppt, wenn die sich gegenüberliegenden Wände der Kerbe 6 aufeinandertreffen, so daß die Kerbe 6 in Längsrichtung und weitgehend in der Tiefenrichtung geschlossen ist. In Fig. 1, die die Rückseite 3 der Prothese zeigt, ist die Kerbe durch eine Vielzahl an Punkten dargestellt.

Wie in Fig. 2 zu sehen ist, hat das Absacken des oberen Teiles 10 des Prothesenkörpers 1 zur Folge, daß der die Brustwarze 14 einschließende mittlere Bereich und der unterhalb der Brustwarze 14 liegende Bereich der Vorderseite 2 des Prothesenkörpers im Vergleich zu der gestrichelt dargestellten, gestreckten Form eine starke Auswölbung erfährt, die mit einer Verschiebung der Brustwarze 14 nach vorne und nach unten einhergeht, wobei der Prothesenrand 4 im unteren Bereich durch den Rand des Körbchens des Büstenhalters am Körper der Trägerin beim Punkt S fixiert wird. Der mittlere Bereich der Vorderseite 2 des Prothesenkörpers ist in der abgesackten Stellung weiter vom Körper der Trägerin entfernt als in der gestreckten Stellung der Prothese. Der Prothesenrand im oberen Bereich der Vorderseite 2 des Prothesenkörpers 1 liegt im zusammengesackten Zustand der Prothese tiefer als bei der gestreckten Stellung der Prothese.

Wechselt die Trägerin von der aufrechten Haltung in eine Liegestellung, ändert die Prothese ihre Form in der Weise, daß sie sich streckt, wie in Fig. 2 dargestellt ist. Unter dem Einfluß der Schwerkraft, die nun um 90° gedreht an der Prothese angreift, wird der obere Teil 10 des Prothesenkörpers wieder nach oben bewegt, wobei die Kerbe 6 eine Form annimmt, wie sie in Fig. 2 strichliniert gezeigt ist. Das Strecken der Prothese wird durch die Rückstellung des elastisch verformten mittleren Abschnitts 12 unterstützt. Als Folge der Streckung der Prothese flacht sich die Vorderseite 2 wieder ab und geht die Wölbung der Vorderseite 2 im mittleren und unteren Bereich zurück. Der tiefste Punkt der Prothese liegt in der gestreckten Stellung im untersten Bereich des Prothesenrandes 4. Die Bewegung der Prothese von der gestreckten in die zusammengesackte Form (und wieder zurück) kann auch als Rollbewegung um den Punkt S beschrieben werden.

Die Kerbe 6 endet im dargestellten ersten Ausführungsbeispiel im Abstand vor dem Prothesenrand 4, wie in Fig. 1 erkennbar ist.

Befindet sich die Trägerin in einer aufrechten Haltung und wird die Prothese einer dynamischen Belastung, beispielsweise beim Laufen, ausgesetzt, ändert die Prothese ihre Form abwechselnd zwischen den in Fig. 2 dargestellten strichlinierten und strichpunktierten Formen. Diese Formänderung unter dynamischer Belastung entspricht weitgehend dem Schwingungsverhalten einer natürlichen Brust bei dynamischer Belastung.

Das abwechselnde Strecken und Zusammensacken der Prothese fördert die Be- und Entlüftung der Rückseite der Prothese, denn durch das sich abwechselnde Öffnen und Schließen der Kerbe 6 wird Luft eingesaugt oder ausgepumpt. Eine gute Be- und Entlüftung der Prothese verhindert eine unangenehme Schweißbildung auf der Haut unter der Prothese.

Im dargestellten Beispiel gemäß Figuren 1 und 2 ist die erfindungsgemäße Prothese eine symmetrische Prothese, d.h., sie kann beidseitig verwendet werden. Die erfinderische Ausbildung der Prothese ist aber auch auf eine asymmetrische Prothese übertragbar, die entweder rechts oder links getragen werden kann. Auf der Rückseite kann ein Stoffteil angebracht werden, welches die Reibung zwischen dem Prothesenkörper und der Haut der Prothesenträgerin verringert. Die Anpassungsfähigkeit der Prothese an die Form der natürlichen Brust beim Liegen und in aufrechter Haltung der Trägerin wird dadurch erhöht.

Die Silikonkautschukmasse der erfindungsgemäßen Prothese hat einen sehr weichen, trägen Charakter. Er wird dadurch erreicht, daß als Silikonkautschukmasse ein additionsvernetzendes Zweikomponenten-Silikonsystem aus α, ω-Dimethylpolydimethylsiloxanen, α, ω-Divinylpolydimethylsiloxanen mit einem Si-Vinyl-Gehalt zwischen 0,01 mmol/g und 0,2 mmol/g, SiH-funktionellen Polydimethylsiloxanen mit einem SiH-Gehalt von 2 mmol/g bis 5 mmol/g und Platinkomplexverbindungen als Katalysatoren verwendet wird. Die Viskosität der beiden Komponenten liegt zwischen 500 bis 2500 mPa.s. Die Weichheit des Zweikomponenten-Silikonsystems wird derart eingestellt, daß die Penetration zwischen 150 bis 230.1/10 mm liegt, wobei die Messung nach DIN 51580 mit einer Masse des Prüfkonus von 14,9 g und der der Konusführungsstange von 10 g erfolgt.

Die elastische Biegeformbarkeit der Prothese im Bereich der Kerbe 6 wird durch den sehr weichen Charakter der Silikonkautschukmasse unterstützt.

Das zweite Ausführungsbeispiel ist dem ersten Ausführungsbeispiel sehr ähnlich soweit es die Zusammensetzung der Prothese und die Form der Vorderseite der Prothese im unverformten Zustand betrifft. Der einzige Unterschied zwischen den beiden Ausführungsbeispielen liegt in der Form der Kerbe, die beim zweiten Ausführungsbeispiel dazu führt, daß der untere Bereich der Vorderseite der Prothese im zusammengesackten Zustand noch stärker ausgewölbt ist, wobei diese Auswölbung sich stärker auf das Zentrum der Prothesenvorderseite konzentriert und infolgedessen eine ausgeprägte Konusform der Vorderseite der Brustprothese vermittelt.

Aufgrund der weitgehenden Übereinstimmung der beiden Ausführungsbeispiele beschränkt sich die folgende Beschreibung daher auf eine Erläuterung der Form der Kerbe und deren Auswirkungen auf die Form der Vorderseite der Prothese. Es werden die gleichen Bezugszeichen wie beim ersten Ausführungsbeispiel verwendet, wobei lediglich ein Strich hinter der jeweiligen Bezugsziffer hinzugefügt ist. Außerdem werden nur diejenigen Teile bezeichnet, auf die zum besseren Verständnis des Unterschieds zwischen den beiden Ausführungsbeispielen Bezug genommen werden muß.

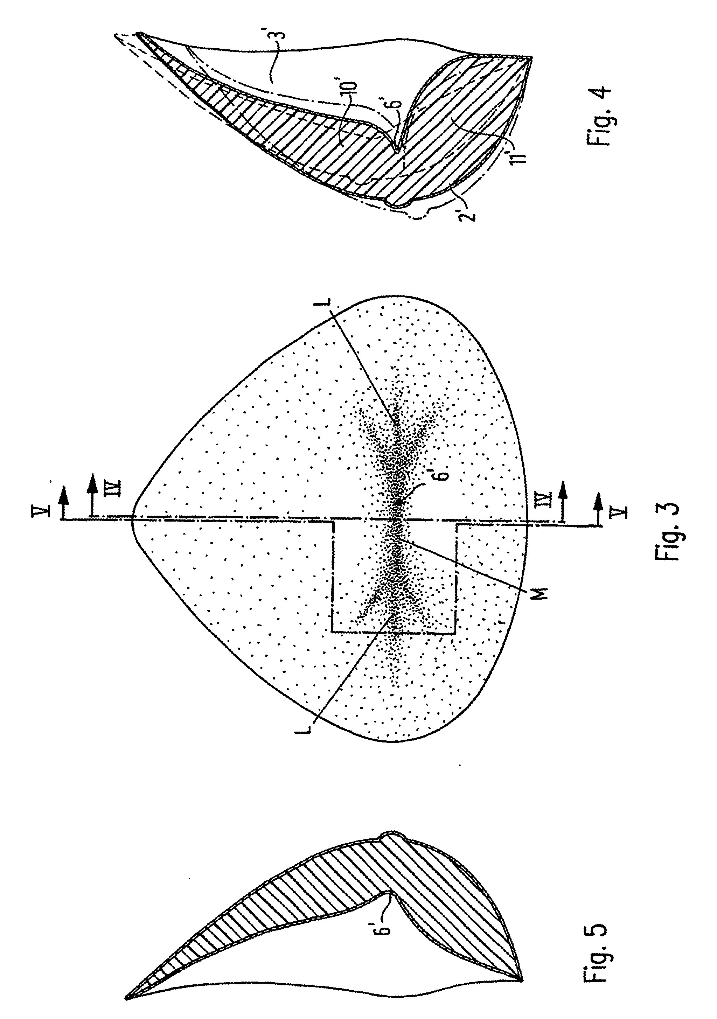

Wie aus der Fig. 3 in Verbindung mit den Figuren 4 und 5 erkennbar ist, hat die Kerbe 6', in Längsrichtung der Kerbe 6' gesehen, einen mittleren Bereich M und zwei seitliche Bereiche L, zwischen denen sich die der mittlere Bereich M befindet. Im mittleren Bereich M der Kerbe 6' sind die sich gegenüberliegenden Wände relativ steil, so daß im mittleren Bereich M die Kerbe 6' relativ eng ist. In den beiden seitlichen Bereichen L der Kerbe 6' sind die sich gegenüberliegenden Wände flacher als im mittleren Bereich M ausgebildet, so daß die Kerbe 6' dort breiter als im mittleren Bereich M ist, aber dennoch im wesentlichen die gleiche Tiefe wie im mittleren Bereich M hat. Außerdem ist im mittleren Bereich M der Kerbe 6' die untere Wand länger leicht gekrümmt nach hinten geführt, bevor sie eine deutliche Richtungsänderung nach unten macht. Im Vergleich dazu macht die obere Wand eine deutliche Richtungsänderung nach oben an einer Stelle, die nicht weit von der tiefsten Stelle der Kerbe 6' entfernt ist. Infolgedessen ist der unterhalb der Kerbe 6' liegende Bereich der Rückseite 3' stärker wulstförmig ausgeprägt als der oberhalb der Kerbe 6' liegende, obere Bereich der Rückseite 3'.

Sackt die Prothese gemäß dem zweiten Ausführungsbeispiel unter der Wirkung der Schwerkraft zusammen, kommen nur die im mittleren Bereich M der Kerbe 6' sich gegenüberliegenden Wände zur Anlage, wogegen sich die Wände der Kerbe 6' in den seitlichen Bereichen L näher kommen aber nicht berühren.. Der obere Teil 10' der Prothese drückt im zusammengesackten Zustand auf eine im Vergleich zum ersten Ausführungsbeispiel verminderte Fläche, die durch die untere Wand der Kerbe 6' in deren mittlerem Bereich M gebildet wird. Dadurch wird das im unteren Teil 11' der Prothese befindliche Material stärker nach unten und nach vorne geschoben, so daß sich auf der Vorderseite 2' eine stärkere Auswölbung in der Mitte der Prothese ergibt.

Aufgrund der steileren Ausführung kommen im mittleren Bereich M der Kerbe 6' die sich gegenüberliegende Wände früher zur Anlage als beim ersten Ausführungsbeispiel. Dies bedeutet, daß das Abbremsen der Absackbewegung mit der daraus resultierenden Verformung der Vorderseite 2' im mittleren und unteren Bereich früher einsetzt als beim ersten Ausführungsbeispiel. Das Strecken der Prothese als Folge eines Wechsels der Trägerin von der aufrechten Haltung in die Liegestellung geht wie beim ersten Ausführungsbeispiel vonstatten.

Anhand der beiden Ausführungsbeispiele wird deutlich, daß die Absackbewegung der Prothese durch unterschiedliche Art der Gestaltung der Kerbe verschieden gesteuert werden kann. Je nach Wahl der Form der Kerbe ergibt sich auch eine unterschiedliche Auswölbung des unteren und mittleren Bereichs der Vorderseite 2' der Prothese.

Auch die Weichheit der Silikonkautschukmasse stellt einen das Absack- und Aufrichtverhalten der Prothese beeinflussenden Parameter dar. Durch Versuche mit verschiedenen Kerbenformen und unterschiedlichen Weichheitsgraden der Silikonkautschukmasse kann somit leicht eine für jede Prothesenträgerin optimal angepaßte Prothese gefunden werden, die höchsten Ansprüchen an Natürlichkeit im Liegen wie im Stand der Trägerin und unter dynamischer Belastung genügt.

## Patentansprüche

1. Brustprothese zum Tragen in einem Büstenhalter oder dergleichen, mit einem Prothesenkörper (1), der eine die Form einer natürlichen Brust aufweisende Vorderseite (2; 2'), eine mit einer Vertiefung (5) versehene Rückseite (3, 3') und einen einer geschlossenen Linie folgenden Prothesenrand (4) hat, an dem die Vorderseite (2; 2') und die Rückseite (3; 3') jeweils enden, wobei der Prothesenkörper (1) eine weich-elastische Silikonkautschukmasse aufweist, die in einen Beutel aus längs des Prothesenrandes (4) dicht miteinander verbundenen, elastisch dehnbaren Kunststoffolien (8, 9) hohlraumfrei eingeschlossen ist, **dadurch gekennzeichnet, daß** die Vertiefung (5) mindestens eine quer zur Körperlängsachse der Trägerin verlaufende Kerbe (6; 6') aufweist, die von sich gegenüberliegenden Wänden eines oberen (10; 10') und eines unteren Teils (11; 11') des Prothesenkörpers (1) und einem mittleren, den oberen mit dem unteren Teil (10, 11; 10', 11') flexibel verbindenden Abschnitt (12) begrenzt wird, der es dem oberen Teil (10; 10') bei aufrechter Haltung der Trägerin gestattet, durch die Wirkung der Schwerkraft relativ zu dem unteren Teil (11; 11') abzusacken, wobei das Absacken des oberen Teils (10; 10') eine elastische Biegeverformung des mittleren Abschnitts (12) bewirkt und durch Anlage zumindest eines Teiles der sich gegenüberliegenden Wände der Kerbe (6; 6') begrenzt wird, und daß sich beim Wechseln von der aufrechten in eine liegende Haltung der Trägerin der obere Teil (10; 10') des Prothesenkörpers (1) sich relativ zu dem unteren Teil (11; 11') nach oben bewegt, wobei sich die Wände der Kerbe (6; 6') voneinander entfernen und die Vorderseite (2; 2) des Prothesenkörpers (1) flacher wird.

2. Brustprothese nach Anspruch 1, **dadurch gekennzeichnet, daß** die Kerbe (6) zumindest im unverformten Zustand einen bogenförmigen Verlauf hat, der die sich gegenüberliegenden Wände der Kerbe (6) zwingt, sich beim Absacken des oberen Teils (10) in Längsrichtung der Kerbe (6) fortschreitend aneinanderzulegen.

3. Brustprothese nach einem der vorhergehenden Ansprüche, **dadurch gekennzeichnet, daß** die Kerbe (6), in Längsrichtung gesehen, im Abstand vor dem Prothesenrand (4) endet.

4. Brustprothese nach einem der vorhergehenden Ansprüche, **dadurch gekennzeichnet, daß** die Vertiefung (3) mehr als eine quer zur Körperlängsachse der Trägerin verlaufende Kerben (6)aufweist, die bezogen auf die Körperlängsachse der Trägerin im Abstand übereinander angeordnet sind.

5. Brustprothese nach Anspruch 1, **dadurch gekennzeichnet, daß** die Kerbe (6') in einem mittleren Bereich (M) steilere Wände als in zwei seitlichen Bereichen (L) hat und nur die Wände im mittleren Bereich (M) der Kerbe (6') zur Anlage miteinander kommen, um die Absackbewegung zu begrenzen.

6. Brustprothese nach einem der vorhergehenden Ansprüche, **dadurch gekennzeichnet, daß** die Silikonkautschukmasse auf eine Weichheit mit einer Penetration zwischen 150 bis 230 eingestellt ist.

7. Brustprothese nach einem der vorhergehenden Ansprüche, **dadurch gekennzeichnet, daß** die Silikonkautschukmasse aus zwei additionsvernetzten Komponenten besteht, deren Viskosität im Bereich zwischen 500 bis 2500 mPa.s liegt.

## Claims

1. A breast prosthesis for wearing in a brassiere or the like, comprising a prosthesis body (1) having a front (2; 2') having the shape of a natural breast, a back (3, 3') provided with a recess (5), and a prosthesis edge (4) which follows a closed line and at which the front (2; 2') and the back (3; 3') respectively end, the prosthesis body (1) comprising a soft-elastic silicone rubber material which is enclosed so as to be free from cavities in a bag consisting of elastically extensible plastic films (8, 9) sealingly interconnected along the prosthesis edge (4), **characterised in that** the recess (5) has at least one groove (6; 6') extending transversely of the longitudinal axis of the wearer's body and which is bounded by oppositely situated walls of a top part (10; 10') and a bottom part (11; 11') of the prosthesis body (1) and a middle section (12) flexibly connecting the top and bottom parts (10, 11; 10', 11'), which section (12) allows the top part (10; 10') to sag in relation to the bottom part (11; 11') under the action of gravity when the wearer is in the upright position, the sagging of the top part (10; 10') resulting in an elastic flexural deformation of the middle section (12) and being limited by at least a part of the oppositely situated walls of the groove (6; 6') coming into contact, and **in that** when the wearer's position changes from the upright position to a reclining position the top part (10; 10') of the prosthesis body (1) moves upwards relatively to the bottom part (11; 11'), the walls of the groove (6; 6') moving away from one another and the front (2; 2') of the prosthesis body (1) becoming flatter.

2. A breast prosthesis according to claim 1, **characterised in that** the groove (6) has at least in the undeformed state an arcuate configuration which forces the oppositely situated walls of the groove (6) to progressively come into contact in the longitudinal direction of the groove (6) during the sagging of the top part (10).

3. A breast prosthesis according to any one of the preceding claims, **characterised in that** the groove (6) as considered in the longitudinal direction terminates at a distance in front of the prosthesis edge (4).

4. A breast prosthesis according to any one of the preceding claims, **characterised in that** the recess (3) has more than one groove (6) extending transversely of the longitudinal axis of the wearer's body, said grooves being disposed one above the other in spaced relationship with respect to the longitudinal axis of the wearer's body.

5. A breast prosthesis according to claim 1, **characterised in that** the groove (6') has steeper walls in a middle zone (M) than in two lateral zones (L) and only the walls in the middle zone (M) of the groove (6') come into contact with one another to limit the sagging movement.

6. A breast prosthesis according to any one of the preceding claims, **characterised in that** the silicone rubber material is adjusted to a softness having a penetration of between 150 and 230.

7. A breast prosthesis according to any one of the preceding claims, **characterised in that** the silicone rubber material consists of two addition crosslinked components, the viscosity of which is in the range between 500 and 2500 mPa.s.

## Revendications

1. Prothèse mammaire pour porter dans un soutien-gorge ou vêtement semblable, avec un élément prothétique (1), qui a une partie antérieure (2 ; 2') de la forme d'un sein naturel, une partie postérieure (3 ; 3') pourvue d'une alvéole (5) et un bord de prothèse (4) suivant une ligne continue auquel aboutissent toutes deux la partie antérieure (2, 2') et la partie postérieure (3, 3'), le corps prothétique (1) présentant une masse de caoutchouc en silicone doux-élastique qui est incorporée sans interstice dans une poche constituée de couches de plastique (8, 9) élastiques extensibles assemblées de façon compacte le long du bord de prothèse (4), **caractérisée en ce que** l'alvéole (5) présente au moins un sillon (6 ; 6') s'étendant transversalement par rapport à l'axe corporel de la porteuse, qui est limité par les parois opposées d'une partie supérieure (10 ; 10') et d'une partie inférieure (11 ; 11') de l'élément prothétique (1) et par une portion moyenne (12) reliant de façon flexible les parties supérieure et inférieure (10, 11 ; 10' ; 11'), qui permet à la partie supérieure (10 ; 10') en cas de position verticale de la porteuse de s'abaisser relativement par rapport à la partie inférieure (11 ; 11') sous l'effet de la gravité, l'abaissement de la partie supérieure (10 ; 10') provoquant une courbure élastique de la portion moyenne (12) et étant limité par la butée d'au moins une partie des parois opposées du sillon (6 ; 6'), et **en ce que**, lors du passage de la position verticale à une position allongée de la porteuse, la partie supérieure (10 ; 10') de l'élément prothétique (1) se soulève relativement vers le haut par rapport à la partie inférieure (11 ; 11'), les parois du sillon (6 ; 6') s'éloignant l'une de l'autre et la partie antérieure (2 ; 2') de l'élément prothétique (1) s'aplatissant.

2. Prothèse mammaire selon la revendication 1, **caractérisée en ce que** le sillon (6) a un tracé voûté au moins à l'état non déformé, qui oblige les parois opposées du sillon (6) à se rapprocher davantage dans le sens de la longueur du sillon (6) lors de l'abaissement de la partie supérieure (10).

3. Prothèse mammaire selon l'une quelconque des revendications, **caractérisée en ce que** le sillon (6), vu dans l'axe de la longueur, se termine à distance du bord de prothèse (4).

4. Prothèse mammaire selon l'une quelconque des revendications, **caractérisée en ce que** l'alvéole (3) présente pas un mais plusieurs sillons (6) s'étendant transversalement par rapport à l'axe corporel de la porteuse et qui sont disposés par rapport à l'axe de la longueur du corps à distance les uns au-dessus des autres.

5. Prothèse mammaire selon la revendication 1, **caractérisée en ce que** le sillon (6') a des parois plus abruptes dans la partie moyenne (M) que dans les deux parties latérales (L) et que seules les parois dans la partie moyenne (M) du sillon (6') viennent en butée pour limiter le mouvement d'abaissement.

6. Prothèse mammaire selon l'une quelconque des revendications précédentes, **caractérisée en ce que** la masse de caoutchouc en silicone est ajustée pour une souplesse avec une pénétration de 150 à 230.

7. Prothèse mammaire selon l'une quelconque des revendications précédentes, **caractérisée en ce que** la masse de caoutchouc en silicone est composée de deux composants polymérisés par addition, dont la viscosité est dans la gamme entre 500 et 2500 mPa.s.
